(19) Europäisches Patentamt
European Patent Office
Office européen des brevets

(11) Veröffentlichungsnummer: **0 331 093**
**A1**

(12) # EUROPÄISCHE PATENTANMELDUNG

(21) Anmeldenummer: **89103450.6**

(22) Anmeldetag: **28.02.89**

(51) Int. Cl.⁴: **C07D 487/04 , A61K 31/505 , //(C07D487/04,239:00,235:00)**

(30) Priorität: **04.03.88 DE 3807084**

(43) Veröffentlichungstag der Anmeldung: **06.09.89 Patentblatt 89/36**

(84) Benannte Vertragsstaaten: **AT BE CH DE ES FR GB IT LI NL**

(71) Anmelder: **KNOLL AKTIENGESELLSCHAFT**
**Knollstrasse**
**D-6700 Ludwighafen(DE)**

(72) Erfinder: **Fernandez Brana, Miguel, Dr.**
**Avenida de Burgos No. 20, 1 M**
**ES-28036 Madrid(ES)**
Erfinder: **Castellano Berlanga, Jose Maria, Dr.**
**Avenida de Burgos No. 20, 3 K**
**ES-28036 Madrid(ES)**
Erfinder: **Redondo Gonzalez, Maria Del Carmen, Dr.**
**Virgen del Castanar No. 7**
**ES-28027 Madrid(ES)**
Erfinder: **Schlick, Erich, Dr.**
**Reiherstrasse 9**
**D-6701 Otterstadt(DE)**
Erfinder: **Keilhauer, Gerhard, Dr.**
**Industriestrasse 20**
**D-6701 Dannstadt-Schauernheim(DE)**

(74) Vertreter: **Karau, Wolfgang Dr. et al**
**BASF Aktiengesellschaft Carl-Bosch-Strasse 38**
**D-6700 Ludwigshafen(DE)**

(54) **Neue Benzimidazo[1,2-c] chinazoline, ihre Herstellung und Verwendung.**

(57) Es werden Benzimidazo[1,2-c]-chinazoline der Formel

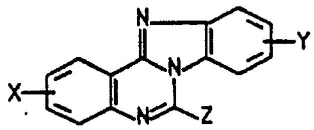

I,

worin X, Y und Z die in der Beschreibung angegebene Bedeutung haben sowie deren Herstellung beschrieben. Die Substanzen eignen sich zur Bekämpfung von Krankheiten.

EP 0 331 093 A1

## Neue Benzimidazo[1,2-c]chinazoline, ihre Herstellung und Verwendung

Die vorliegende Erfindung betrifft neue Benzimidazo[1,2-c]-chinazoline, Verfahren zu deren Herstellung und deren Verwendung in Arzneimitteln mit tumorhemmenden und antiviralen Eigenschaften.

Es sind bereits einige Benzimidazo[1,2-c]-chinazoline bekannt, vgl. Heterocyclic Chem. 3, 289 (1966), ibid. 10, 65 (1973), Ukr. Klim. Zh 45, 225 (1979), Acta Ciencia Indica [Ser.] Chem. 10, 178 (1984), Aust. J. Chem. 38, 1685 (1985), Acta Pharm. Yugosl. 36, 281 (1986). Eine antitumorale oder antivirale Wirkung ist für diese Verbindungen jedoch nicht angegeben worden.

Es wurde nun gefunden, daß Benzimidazo[1,2-c]-chinazoline der Formel I

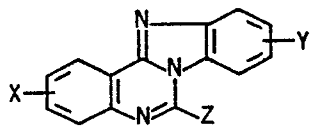

I,

worin

X und Y gleich oder verschieden sind und $C_{1-4}$-Alkyl-, Nitro-, Amino-, Hydroxy-, $C_{1-4}$-Hydroxyalkyl-, Cyano-, $C_{1-4}$-Alkoxy-, $C_{1-4}$-Alkoxycarbonyl-, Carbamoyl-, $C_{1-4}$-Halogenalkyl- oder $C_{1-4}$-Trihalogenalkylgruppen oder Halogenatome darstellen und

Z eine Dialkylaminoalkyl-, Cycloalkylidenaminoalkyl-, Dialkylaminoalkylidenamin- oder Cycloalkylidenaminoalkylidenaminogruppe, die in den Alkyl- bzw. Alkylidengruppen jeweils bis zu 4 C-Atome und in den Cycloalkylidengruppen 2 bis 5 C-Atome besitzen, darstellt,

sowie deren Salze mit physiologisch verträglichen Säuren eine günstige Wirkung besitzen.

Bevorzugt sind die Verbindungen, in denen X und Y Amino- oder Nitrogruppen sind und die Alkyl- bzw. Alkylidengruppen in Z bis zu 2 C-Atome besitzen sowie solche, deren Cycloalkylidenaminogruppe ein Pyrrolidinrest ist.

Als physiologisch verträgliche Säuren eignen sich zur Salzbildung organische und anorganische Säuren, wie Salzsäure, Schwefelsäure, Phosphorsäure, Essigsäure, Zitronensäure, Oxalsäure, Malonsäure, Salizylsäure, Maleinsäure, Fumarsäure, Bernsteinsäure, Ascorbinsäure, Äpfelsäure, Methansulfonsäure, Isethionsäure, Milchsäure, Gluconsäure, Glucuronsäure, Amidosulfonsäure, Benzoesäure, Weinsäure, Pamoasäure.

Die neuen Verbindungen können in solvatisierter Form vorliegen. Solche Formen können sich beispielsweise mit Wasser oder Ethanol bilden.

Die neuen Verbindungen werden hergestellt, indem man

a) - falls Z eine Dialkylaminoalkylgruppe darstellt -

α) aus einer Verbindung der Formel II

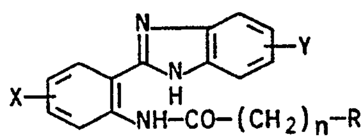

II,

worin

X und Y die angegebene Bedeutung haben,

R eine Di-$C_{1-4}$-Alkylamino- oder Cyclo-$C_{2-5}$-alkylidenaminogruppe ist,

n die Zahl 1, 2, 3 oder 4 bedeutet, Wasser abspaltet oder

β) eine Verbindung der Formel III

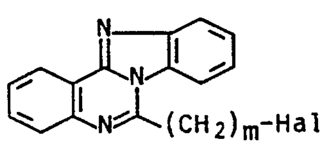

III,

worin

m.die Zahl 1, 2, 3 oder 4 bedeutet,
mit einem Di-$C_{1-4}$-alkylamin oder Cyclo-$C_{2-5}$-alkylidenamin umsetzt oder

b) - falls Z eine Dialkylaminoalkylidenamino- oder Cycloalkylidenaminoalkylidenaminogruppe darstellt - eine Verbindung der Formel IV

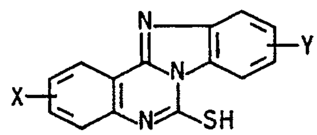

IV,

worin
X und Y die angegebene Bedeutung besitzen,
mit einem Dialkylaminoalkylidendiamin umsetzt oder Cycloalkylidenaminoalkylidenamin umsetzt und die so erhaltenen Verbindungen anschließend gegebenenfalls in ihre Salze mit physiologisch verträglichen Säuren überführt.

Die Umsetzung gemäß a) α) wird zweckmäßig mit Hilfe eines anorganischen Säurehalogenids in Gegenwart einer Base bei Raumtemperatur oder erhöhter Temperatur durchgeführt. Zweckmäßig arbeitet man in Gegenwart eines Lösungsmittels.

Die Reaktion a) β) erfolgt durch Zugabe des Dialkylamins zur Halogenverbindung III. Entsprechendes gilt für die Umsetzung gemäß b), jedoch arbeitet man zweckmäßig bei erhöhter Temperatur.

Die für die Reaktionen benötigten Ausgangsstoffe sind bekannt und können, wie im experimentellen Teil unter I beschrieben, hergestellt werden.

Die neuen Verbindungen eignen sich zur Behandlung von festen Tumoren, wie Carcinome in Mamma-, Lungen-, Colon- und Nierenbereich, sowie von akuter und chronischer Leukämie sowie viralen Erkrankungen.

Die erfindungsgemäßen Verbindungen können in üblicher Weise oral oder parenteral verabfolgt werden. Sie können in den gebräuchlichen galenischen Applikationsformen fest oder flüssig angewendet werden, z.B. als Tabletten, Filmtabletten, Kapseln, Granulate, Dragees oder Lösungen. Diese werden in üblicher Weise hergestellt. Die Wirkstoffe können dabei mit den üblichen galenischen Hilfsmitteln wie Tablettenbindern, Füllstoffen, Konservierungsmitteln, Tablettensprengmitteln, Fließreguliermitteln, Weichmachern, Netzmitteln, Dispergiermitteln, Emulgatoren, Lösungsmitteln, Retardierungsmitteln und/oder Antioxidantien verarbeitet werden (vgl. H. Sucker et al: Pharmazeutische Technologie, Thieme-Verlag, Stuttgart, 1978). Die so erhaltenen Applikationsformen enthalten den Wirkstoff normalerweise in einer Menge von 10 bis 90 Gew.-%.

## Beispiele

### I. Herstellung der Ausgangsmaterialien

A. Für das Verfahren a) α):

1a. Eine Lösung von 5,3 g (0,047 Mol) Chloracetylchlorid in 10 ml Chloroform wurde tropfenweise und unter Rühren in eine Suspension von 10 g (0,047 Mol) 2-(2-Aminophenyl)benzimidazol in 125 ml Chloroform gegeben. Die Mischung wurde 3 h bei Raumtemperatur gerührt. Der entstandene Niederschlag wurde abfiltriert, mit einer 5%igen Natriumhydrogencarbonat-Lösung gewaschen und aus Ethanol umkristallisiert. Man erhielt 10 g (75 %) 2-(2-Chloracetamidophenyl)benzimidazol, F = 170° C.

Analog wurden hergestellt:

1b. 2-(2-Chloracetamido-5-methylphenyl)benzimidazol, Ausbeute: 30 %, F = 315° C (Ethanol)
1c. 2-[2-(3-Chlorpropionamido)-phenyl]benzimidazol, Ausbeute 67 %, F = 170 - 173° C (Ethanol)

1d. 2-[2-(3-Chlorpropionamido)-methylphenyl]benzimidazol, Ausbeute 64 %, F = 174° C (Ethanol)

1e. 2-[2-(2-Chloracetamido)-5-chlorphenyl]benzimidazol, Ausbeute 55 %, F = 260 - 262° C (Dimethylformamid/Wasser)

1f. 2-[2-(3-Chlorpropionamido)-5-chlorphenyl]benzimidazol, Ausbeute 75 %, F = 233 - 235° C.

1g. 2-[2-(4-Chlorbutyramido)-5-chlorphenyl]benzimidazol, Ausbeute 50 %, F = 156 - 158° C (Ethanol/Wasser)

1h. 2-[2-(3-Chlorpropionamido)-phenyl]benzimidazol Ausbeute 60 %, F = 229 - 230° C (Ethanol/Wasser)

1i. 2-[2-(3-Chlorpropionamido)-4-nitrophenyl]benzimidazol, Ausbeute 79 %, F = 204 - 206° C (Aceton)

1j. 2-[2-(4-Chlorbutyramido)-4-nitrophenyl]benzimidazol Ausbeute 57 %, F = 161 - 163° C (Aceton).

2a. Eine Mischung aus 3 g (0,01 Mol) 1c und 30 ml Dimethylamin (40%ige wäßrige Lösung) wurde 24 h bei Raumtemperatur gerührt. Überschüssiges Amin wurde unter Unterdruck abgezogen und der Rückstand mit Wasser versetzt, wobei sich ein Niederschlag bildete. Dieser wurde abgesaugt und aus Toluol/Cyclohexan umkristallisiert. Man erhielt 3 g (97 %) 2-[2-(3-Dimethylaminopropionamido)-phenyl]-benzimidazol, F = 162 -163° C.

Analog wurde hergestellt:

2b. 2-[2-(3-Dimethylaminopropionamido)-5-methylphenyl]-benzimidazol, Ausbeute 51 %, F = 233 - 235° C (Ethanol)

2c. 2-[2-(2-Dimethylaminoacetamido)-5-chlorphenyl]benzimidazol, Ausbeute 52 %, F = 223 - 225° C (Ethanol)

2d. 2-[2-(3-Dimethylaminopropionamido)-5-chlorphenyl]benzimidazol, Ausbeute 71 %, F = 180 - 182° C (Dimethylformamid/Wasser)

2e. 2-[2-(3-Dimethylaminopropionamido)-4-chlorphenyl]-benzimidazol, Ausbeute 60 %, F = 194° C (Ethanol/Wasser)

2f. 2-[2-(3-Dimethylaminopropionamido)-4-nitrophenyl]-benzimidazol, Ausbeute 74 %, F = 210 - 212° C (Dimethylformamid/Wasser)

2g. 2-[2-(4-Dimethylaminobutyramido)-4-nitrophenyl]benzimidazol, Ausbeute 64 %, F = > 250° C (Aceton)

3a. Eine Mischung aus 2,28 g (0,0073 Mol) 1d, 15 ml Ethanol und 3 ml Pyridin wurde 4 h unter Rückfluß gekocht. Die Reaktionsmischung wurde abgekühlt. Nach Stehen über Nacht waren 1,2 g (48 %) 2-[2-[3-(1-Pyrrolidin)propionamido]-5-methylphenyl]benzimidazol in Form weißer Kristalle ausgefallen, F = 120 bis 122° C (Ethanol).

3b. 2-[2-[2-(1-Pyrrolidin)acetamido]-5-chlorphenyl]benzimidazol, Ausbeute 50 %, F = 200 - 202° C (Ethanol)

3c. 2-[2-[2-(1-Pyrrolidin)propionamido]-5-chlorphenyl]benzimidazol, Ausbeute 81 %, F = 184 - 186° C (Ethanol)

B. Für das Verfahren a) $\beta$)

Eine Mischung aus 6,4 g (0,022 Mol) 1a und 0,5 g Schwefelsäure in 150 ml Toluol wurde 5 h unter Rückfluß gekocht, wobei das entstehende Wasser azeotrop abdestilliert wurde. Nach dem Abkühlen des Gemisches wurde das Lösungsmittel im Vakuum abgezogen. Nach Zugabe von 50 ml Chloroform wurde die Mischung filtriert und der feste Rückstand aus Toluol/Petrolether umkristallisiert. Es wurden 7,1 g (70 %) 6-Chlormethylbenzimidazo[1,2-c]chinazolin erhalten, F = 224 - 225° C.

C. Für das Verfahren b):

4a. Eine Mischung aus 3,1 g (0,0147 Mol) 2-(2-Aminophenyl)-benzimidazol, 1,5 g (0,026 Mol) Schwefelkohlenstoff, 50 ml Ethanol und 1 g Kaliumhydroxid in 6 ml Wasser wurde 3 h auf einem Wasserbad unter Rückfluß gehalten. Überschüssiger Schwefekohlenstoff und Ethanol wurde unter reduziertem Druck abgezogen und der Rest mit Essigsäure gewaschen und aus Ethanol umkristallisiert. Man erhielt

2 g (54 %) 6-Mercaptobenzimidazo[1,2-c]-chinazolin, F = 303 - 305 °C.

Analog wurden hergestelt:

4b. 2-Methyl-6-mercaptobenzimidazo[1,2-c]chinazolin, Ausbeute 46 %, F = 321 - 323 °C (Ethanol)

4c. 2-Chlor-6-mercaptobenzimidazo[1,2-c]chinazolin, Ausbeute 45 %, F = 322 - 324 °C (Dimethylformamid/Wasser)

4d. 3-Chlor-6-mercaptobenzimidazo[1,2-c]chinazolin, Ausbeute 57 %, F = >350 °C (Dimethylformamid/Wasser)

4e. 3-Nitro-6-mercaptobenzimidazo[1,2-c]chinazolin, Ausbeute 48 %, F = >350 °C (Dimethylformamide/Wasser)

II. Herstellung der Endprodukte

Beispiel 1

Zu einer Lösung von 2,5 g (0,008 Mol) 2-[2-(3-Dimethylaminopropionamido)phenyl]benzimidazol (2a) und Pyridin in 43 ml alkoholfreiem Chloroform wurden unter Rühren 11,52 g (0,096 Mol) Thionylchlorid getropft. Nach 24 h Rühren wurde das Lösungsmittel im Vakuum abgezogen und der Rückstand mit 100 ml 10%iger Natronlauge gewaschen. Anschließend wurde der Rückstand abfiltriert und aus Cyclohexan umkristallisiert. Man erhielt 2 g (87 %) 6-Dimethylaminoethylbenzimidazo[1,2-c]chinazolin, F = 112 °C.

Analog wurden hergestellt:

Beispiel 2

2-Methyl-6-dimethylaminomethylbenzimidazo[1,2-c]chinazolin, Ausbeute 65 %, F = 163 - 165 °C (Cyclohexan)

Beispiel 3

2-Methyl-6-[2-(1-pyrrolidin)ethyl]benzimidazo[1,2-c]chinazolin, Ausbeute 89 %, F = 147 - 149 °C (Cyclohexan)

Beispiel 4

2-Chlor-6-(2-dimethylaminoethyl)benzimidazo[1,2-c]chinazolin, Ausbeute 75 %, F = 118 °C (Cyclohexan)

Beispiel 5

3-Chlor-6-(2-dimethylaminoethyl)benzimidazo[1,2-c]chinazolin, Ausbeute 25 %, F = 194 - 196 °C (Cyclohexan)

Beispiel 6

3-Nitro-6-(3-dimethylaminopropyl)benzimidazo[1,2-c]chinazolin, Ausbeute 66 %, F = 205 - 207 °C (Cyclohexan)

Beispiel 7

Zu einer Lösung von 0,51 g (0,0014 Mol) 2-[2-[3-(1-pyrrolidin)-propionamido]-5-chlorphenyl]-benzimidazol (3c) in 30 ml Toluol wurden 0,37 g Kieselgel 60 zugegeben. Zu der Suspension wurden bei 80° C 0,3 ml Phosphoroxychlorid zugegeben. Danach wurde die Mischung noch 1,5 h bei 80° C gehalten. Nach dem Abkühlen wurde abdekantiert und der Rückstand in 50 ml Wasser unter Zugabe von 25%iger Natronlauge (ad pH 10) suspendiert. Die Suspension wurde filtriert und der Rückstand 4x mit 25 ml heißem Ethanol extrahiert, wobei das Kieselgel zurückblieb. Die Ethanol-Lösungen wurden zur Trockene eingeengt. und der Rückstand aus Cyclohexan umkristallisiert. Man erhielt 100 mg (20 %) 2-Chlor-6-[2-(1-pyrrolidin)-ethyl]benzimidazo[1,2-c]chinazolin, F = 140 -142° C.

Analog wurde hergestellt:

Beispiel 8

3-Nitro-6-[2-dimethylaminoethyl]benzimidazo[1,2-c]chinazolin, Ausbeute 39 %, F = 208° C (Cyclohexan).

Beispiel 9

Eine Mischung aus 2,7 g (0,01 Mol) 6-Chlormethylbenzimidazo[1,2-c]chinazolin (B) und 25 ml 40%igem Dimethylamin in Wasser wurde 48 h bei Raumtemperatur gerührt. Der entstandene Niederschlag wurde abfiltriert, mit Wasser gewaschen und aus Cyclohexan umkristallisiert. Man erhielt 2 g (73 %) 6-Dimethylaminomethylbenzimidazo[1,2-c]chinazolin, F = 135 - 137° C.

Beispiel 10

Eine Lösung von 0,5 g (0,002 Mol) 6-Mercaptobenzimidazo[1,2-c]chinazolin (4a) in 3 ml N,N-Dimethylethylendiamin wurde 6 h unter Rückfluß gekocht. Nach Abkühlen der Mischung wurden 60 ml Wasser zugesetzt und der Niederschlag abfiltriert und aus Cyclohexan umkristallisiert. Man erhielt 0,48 g (73 %) 6-[2-(Dimethylamino)ethylamino]benzimidazo[1,2-c]chinazolin, F = 137 - 139° C.

Analog wurde hergestellt:

Beispiel 11

6-[2-(1-Pyrrolidin)ethylamino]benzimidazo[1,2-c]chinazolin, Ausbeute 70 %, F = 112 - 113° C (Cyclohexan)

Beispiel 12

2-Methyl-6-[2-(dimethylamino)ethylamino]benzimidazo[1,2-c]chinazolin, Ausbeute 77 %, F = 167 - 169° C (Cyclohexan)

Beispiel 13

2-Methyl-6-[2-(1-pyrrolidin)ethylamino]benzimidazo[1,2-c]chinazolin, Ausbeute 72 %, F = 155 - 156° C (Cyclohexan)

Beispiel 14

2-Chlor-6-[2-(dimethylamino)ethylamino]benzimidazo[1,2-c]chinazolin, Ausbeute 76 %, F = 196 - 198° C (Cyclohexan)

Beispiel 15

3-Chlor-6-[2-(dimethylamino)ethylamino]benzimidazo[1,2-c]chinazolin, Ausbeute 55 %, F = 162 - 164° C (Cyclohexan)

Beispiel 16

3-Amino-6-[2-(2-(dimethylamino)ethylamino]benzimidazo[1,2-c]chinazolin, Ausbeute 80 %, F = 168 - 170° C (Cyclohexan)

## Ansprüche

1. Benzimidazo[1,2-c]chinazoline der Formel I

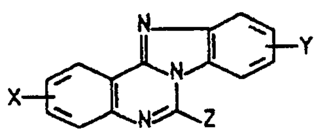

I,

worin
X und Y gleich oder verschieden sind und $C_{1-4}$-Alkyl-, Nitro-, Amino-, Hydroxy-, $C_{1-4}$-Hydroxyalkyl-, Cyano-, $C_{1-4}$-Alkoxy-, $C_{1-4}$-Alkoxycarbonyl-, Carbamoyl-, $C_{1-4}$-Halogenalkyl- oder $C_{1-4}$-Trihalogenalkylgruppen oder Halogenatome darstellen, und
Z eine Dialkylaminoalkyl-, Cycloalkylidenaminoalkyl-, Dialkylaminoalkylidenamino- oder Cycloalkylidenaminoalkylidenaminogruppe, die in den Alkyl- bzw. Alkylidengruppen jeweils bis zu 4 C-Atome und in den Cycloalkylidengruppen 2 bis 5 C-Atome besitzen, darstellt,
sowie deren Salze mit physiologisch verträglichen Säuren.

2. Verfahren zur Herstellung der Benzimidazo[1,2-c]chinazoline der Formel I gemäß Anspruch 1, dadurch gekennzeichnet, daß man

a) - falls Z eine Dialkylaminoalkyl- oder Cycloalkylidenaminoalkylidengruppe darstellt -

α) aus einer Verbindung der Formel II

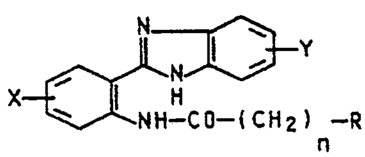

II,

worin
X und Y die angegebene Bedeutung haben und R eine Di-$C_{1-4}$-alkylamino- oder Cyclo-$C_{2-5}$-alkylidenaminogruppe ist,
n die Zahl 1, 2, 3 oder 4 bedeutet, Wasser abspaltet oder
β) eine Verbindung der Formel III

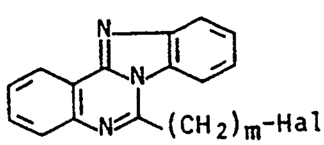

III,

worin
m die Zahl 1, 2, 3 oder 4 bedeutet,
mit einem Di-$C_{1-4}$-alkylamin oder Cyclo-$C_{2-5}$-alkylidenamin umsetzt oder

b) - falls Z eine Dialkylaminoalkylidenamino- oder Cycloalkylidenaminoalkylidenaminogruppe darstellt -eine Verbindung der Formel IV

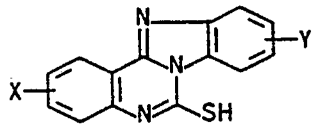

IV,

worin
X und Y die angegebene Bedeutung besitzen, mit einem Dialkylaminoalkylidendiamin oder Cycloalkylidenaminoalkylidenamin umsetzt und die so erhaltenen Verbindungen anschließend gegebenenfalls in ihre Salze mit physiologisch verträglichen Säuren überführt.

3. Benzimidazo[1,2-c]chinazoline der Formel I gemäß Anspruch 1 zur Verwendung bei der Bekämpfung von Krankheiten.

4. Verwendung der Benzimidazo[1,2-c]chinazoline der Formel I gemäß Anspruch 1 bei der Bekämpfung von Tumoren, Leukämie und/oder viralen Erkrankungen.

Europäisches Patentamt

# EUROPÄISCHER RECHERCHENBERICHT

Nummer der Anmeldung

EP 89103450.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | CHEMICAL ABSTRACTS, Band 106, Nr. 5, 02. Februar 1987<br>L.N.VOSTROVA et al. "Synthesis and biological activity of substituted benzimidazo [1,2-c] quinazolines" Seite 544, Spalte 1, Zusammenfassung-Nr. 32 976w<br>& KHIM-FARM. 2H. 1986, 20(6), 690-3<br>-- | 1-3 | C 07 D 487/04<br>A 61 K 31/505/<br>(C 07 D 487/04<br>C 07 D 239:00<br>C 07 D 235:00) |
| D,A | CHEMICAL ABSTRACTS, Band 106, Nr. 21, 25. Mai 1987<br>V.K.PANDEY et al. "Synthesis of some benzimidazoquinazolines as possible antifertility agents" Seite 85, Spalte 2, Zusammenfassung-Nr. 169 230t<br>& ACTA PHARM. JUGOSL. 1986, 36(3) 281-7<br>-- | 1,3 | **RECHERCHIERTE SACHGEBIETE (Int. Cl.4)** |
| A | EP - A2 - 0 241 795 (BAYER AG)<br>* Ansprüche 1,6 *<br>---- | 1,2 | C 07 D 487/00 |

Der vorliegende Recherchenbericht wurde für alle Patentansprüche erstellt.

| Recherchenort | Abschlußdatum der Recherche | Prüfer |
|---|---|---|
| WIEN | 22-05-1989 | PETROUSEK |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82